(19) Europäisches Patentamt
European Patent Office
Office européen des brevets

(11) Veröffentlichungsnummer: **0 213 440 B1**

(12) # EUROPÄISCHE PATENTSCHRIFT

(45) Veröffentlichungstag der Patentschrift: **19.09.90**

(51) Int. Cl.[5]: **C07C 69/00**, C07C 67/00, C07D 317/00

(21) Anmeldenummer: **86110847.0**

(22) Anmeldetag: **05.08.86**

(54) **Polymerisierbare Verbindungen und Verfahren zu ihrer Herstellung.**

(30) Priorität: **13.08.85 DE 3528928**

(43) Veröffentlichungstag der Anmeldung:
**11.03.87 Patentblatt 87/11**

(45) Bekanntmachung des Hinweises auf die Patenterteilung:
**19.09.90 Patentblatt 90/38**

(84) Benannte Vertragsstaaten:
**AT BE CH DE FR GB IT LI NL SE**

(56) Entgegenhaltungen:
**FR-A-1 179 804**
**US-A-3 152 146**
**US-A-3 629 197**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT, Postfach 80 03 20, D-6230 Frankfurt am Main 80(DE)**

(72) Erfinder: **Sander, Jürgen, Dr. Dipl.-Chem., Eichkopfallee 27, D-6237 Liederbach(DE)**
Erfinder: **Schneller, Arnold, Dr. Dipl.-Chem., Asternweg 41, D-6500 Mainz 21(DE)**

EP 0 213 440 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft polymerisierbare Ester der Acryl- oder Methacrylsäure mit mehrwertigen Phenolen sowie ein Verfahren zu ihrer Herstellung.

Monoacrylate und -methacrylate von zweiwertigen Phenolen sind in den DE-A 19 33 657 und 26 59 809 beschrieben. Zu ihrer Herstellung werden zweiwertige Phenole z. B. mit Acryl- bzw. Methacrylsäurechlorid umgesetzt. Die dabei entstehenden Reaktionsgemische enthalten neben unumgesetzten Ausgangsverbindungen mono- und diacylierte Phenole, die sich nur schwierig und unter Ausbeuteverlusten trennen lassen. Die isolierten Monoester enthalten in der Regel noch die angegebenen Verunreinigungen und werden in Ausbeuten erhalten, die insgesamt deutlich unterhalb 50 %, bezogen auf die Ausgangsstoffe, liegen. Die Verunreinigungen durch Verbindungen mit mehr als einer polymerisierbaren Estergruppe im Molekül können bei der Weiterverarbeitung der Verbindungen zu Polymerisaten zu erheblichen Störungen führen, da durch sie verzweigte und ggf. vernetzte Polymerisate entstehen, die ganz andere Löslichkeits- und Viskositätseigenschaften aufweisen als lineare Polymerisate.

Die FR-A 1 179 804 (= GB-A 812 673) beschreibt anionische Carbonate von Dihydroxyphenolen, die in Verbindung mit Gelatine und einer Silberhalogeniddispersion zu einem lichtempfindlichen Film verarbeitet werden. Die Verbindungen können nicht weiter mit einem Acryl- bzw. Methacrylsäurechlorid umgesetzt werden, da keine freien OH-Gruppen zur Verfügung stehen.

Aufgabe der Erfindung war es, Monoacrylate und -methacrylate von mehrwertigen Phenolen in reiner Form bereitzustellen sowie ein Verfahren vorzuschlagen, nach dem diese Verbindungen in reiner Form und hoher Ausbeute erhältlich sind.

Gegenstand der Erfindung ist eine Verbindung der allgemeinen Formel I

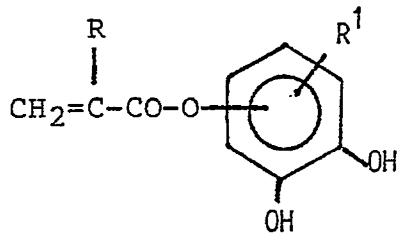

(I)

worin

R ein Wasserstoff- oder Halogenatom, eine Cyanid- oder eine Alkylgruppe mit 1-4 Kohlenstoffatomen und

$R^1$ ein Wasserstoff- oder Halogenatom, eine Nitro-, Alkyl-, Alkoxy-, Aryl-, Aryloxy-, Acyl- oder Alkoxycarbonylgruppe ist.

Erfindungsgemäß wird weiterhin ein Verfahren zur Herstellung von Verbindungen der Formel I vorgeschlagen, das darin besteht, daß man eine Verbindung der Formel II

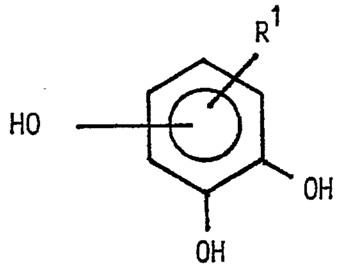

(II)

in der $R^1$ die oben angegebene Bedeutung hat, mit einer Verbindung der Formel III

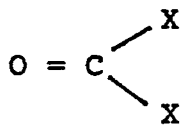

(III)

worin X ein Halogenatom, eine Alkoxy- oder Aryloxygruppe bedeutet, zu einer Verbindung der Formel IV

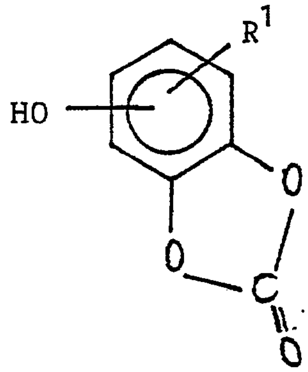

(IV)

umsetzt, die erhaltene Verbindung der Formel IV mit einer Verbindung der Formel V

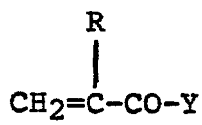

(V)

worin
Y ein Halogenatom, eine Hydroxygruppe, eine Alkoxygruppe oder ein Rest der Formel

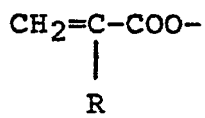

ist und
R die oben angegebene Bedeutung hat,
zu einer Verbindung der Formel VI

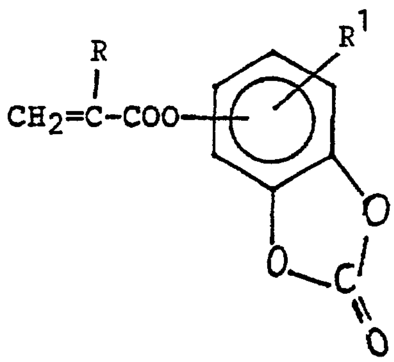

(VI)

umsetzt und die erhaltene Verbindung der Formel VI zu einer Verbindung der Formel I hydrolysiert.

Wenn in der Formel I R eine Alkylgruppe ist, hat diese vorzugsweise 1 oder 2 Kohlenstoffatome, besonders bevorzugt werden Verbindungen mit R = H oder Methyl.

Wenn $R^1$ ein Halogenatom ist, kann es vorzugsweise ein Fluor-, Chlor- oder Bromatom, insbesondere ein Chloratom sein. Wenn $R^1$ eine Alkyl- oder Alkoxygruppe ist, hat diese bevorzugt 1 bis 6, insbesondere 1 bis 3 Kohlenstoffatome; als Acyl- und Alkoxycarbonylgruppen werden solche mit 2 bis 15, insbesondere 2 bis 10 Kohlenstoffatomen bevorzugt. Aryl- und Aryloxygruppen $R^1$ haben im allgemeinen 6 bis 10 Kohlenstoffatome. Der Rest $CH_2=C(R)-COO$ kann in 3- oder 4-Stellung zu den beiden phenolischen Hydroxygruppen stehen, besonders bevorzugt ist die 3-Stellung. $R^1$ kann ebenfalls in 3- oder 4-Stellung stehen.

Beispiele für Verbindungen der Formel II sind Hydroxyhydrochinon, 5-Ethyl-pyrogallol, Pyrogallol, Gallussäuremethylester, Gallussäureethylester, Gallussäurepropylester, Gallussäureoctylester und Gallussäuredodecylester. Verbindungen der Formel II können durch übliche Halogenierungs- oder Nitrierungsverfahren außerdem leicht in die entsprechenden ein-, zwei- oder dreifach substituierten Chlor-, Brom-, Jod- oder Nitro-Derivate übergeführt werden.

Als Vertreter der Verbindungen der Formel III sind Phosgen, Diethylcarbonat oder Diphenylcarbonat zu nennen. Beim Einsatz von Diphenylcarbonat ist kein Lösungsmittel erforderlich. Durch Erhitzen auf

etwa 200-300°C bilden sich 2 mol Phenol, die bei diesen Reaktionsbedingungen abdestillieren. Die Reinigung der zurückbleibenden cyclischen Carbonate der Formel IV erfolgt zunächst durch Vakuumdestillation, anschließend i.a. durch Umkristallisation.

Bei Einsatz von Phosgen wird bevorzugt ein unpolares Lösungsmittel, z. B. Toluol oder Xylol, verwendet. Phosgen wird üblicherweise unter Eiskühlung in Gegenwart von 2 mol einer Base wie Pyridin, Dimethylanilin oder Triethylamin eingeleitet. Nach der üblichen Aufarbeitung mit Säure und Wasser können ebenfalls Verbindungen der Formel IV durch Entfernen des Lösungsmittels erhalten und durch Umkristallisation gereinigt werden.

Die Verbindungen II und III werden zumeist im Molverhältnis 0,8:1,2 bis 1,2:0,8, bevorzugt 0,9:1,1 bis 1,1:0,9 miteinander umgesetzt. Werden Verbindungen der Formel II mit Kohlensäurederivaten der Formel III in einem abweichenden Molverhältnis umgesetzt, so sinken die Ausbeuten, da bei Unterschuß an eingesetztem Kohlensäurederivat noch unumgesetztes Ausgangsmaterial vorhanden ist und bei Überschuß an eingesetztem Kohlensäurederivat die freie Hydroxygruppe von Reaktionsprodukten der Formel IV mit dem überschüssigen Reagens zu intra- oder inter-molekularen Nebenprodukten weiterreagiert.

Bevorzugte Vertreter der Verbindungen der Formel V sind Acrylsäurechlorid, Methacrylsäurechlorid oder Methacrylsäureanhydrid. Weitere Vertreter sind z. B. Acrylsäure, Methacrylsäure, 2-Ethylacrylsäure und 2-Butylacrylsäure sowie deren Methyl- oder Ethylester.

Die Veresterung der Verbindung IV muß so durchgeführt werden, daß die Carbonatgruppierung als Schutzgruppe erhalten bleibt. Dies wird erreicht, wenn man z. B. Acryl- oder Methacrylsäurechlorid in aprotischen Lösungsmitteln wie Chlorkohlenwasserstoffen, z. B. Methylenchlorid, Chloroform, Tetrachlorkohlenstoff, oder aromatischen Kohlenwasserstoffen, z. B. Toluol oder Xylol, in Gegenwart von Basen wie Triethylamin umsetzt. Man kann auch vorteilhaft das billigere Methacrylsäureanhydrid an Stelle von Methacrylsäurechlorid verwenden. Dies kann in Gegenwart von Basen oder auch in Gegenwart von Säuren, z. B. von konzentrierter Schwefelsäure, erfolgen. Bei Verwendung von Acrylsäure und Methacrylsäure oder deren Methyl- bzw. Ethylestern können auch direkte Ver- oder Umesterungen unter sauren oder neutralen Bedingungen erfolgen.

Die auf diese Weise hergestellten Reaktionsprodukte der Formel VI sind i. a. kristallin und können leicht durch Umkristallisieren gereinigt werden. Sie werden dann vorzugsweise in Wasser im Temperaturbereich von 50-100°C hydrolysiert. Dabei werden die beiden als cyclisches Carbonat geschützten Hydroxygruppen freigesetzt.

Die Abspaltung der Carbonatschutzgruppe muß so erfolgen, daß die ungesättigte Estergruppierung erhalten bleibt. Dies wird in einer bevorzugten Variante in neutralem wäßrigem Medium erreicht. Dabei hat es sich als vorteilhaft erwiesen, die zur Hydrolyse eingesetzte Menge Wasser so zu regulieren, daß sich in der Hitze eine klare Lösung bildet und nach dem Abkühlen die Verbindungen der Formel I vollständig auskristallisieren. Sie werden nach diesem Verfahren mit einer Gesamtausbeute von wenigstens 50 % erhalten. Das beschriebene Verfahren kann auf alle Trihydroxyaromaten, die zwei alphaständige Hydroxygruppen aufweisen, angewendet werden.

Die neuen Monomeren können zur Herstellung von Polymeren in der üblichen Weise mittels Wärme, Strahlung und Katalysatoren, z. B. Peroxiden oder Azoverbindungen, entweder homopolymerisiert oder auch mit anderen ethylenisch ungesättigten Monomeren mischpolymerisiert werden.

Die Verarbeitung der erfindungsgemäßen Verbindungen zu Polymeren und deren Verwendung als Bindemittel in lichtempfindlichen Gemischen ist in der gleichzeitig eingereichten Patentanmeldung P 35 28 930.9 beschrieben.

Die folgenden Herstellungsbeispiele erläutern die Erfindung.

Beispiel 1

Herstellung von 1,2-Dihydroxy-3-methacryloyloxy-benzol

In einem Kolben mit Destillationsaufsatz wurden 504 g Pyrogallol und 816 g Diphenylcarbonat gut vermischt und allmählich auf 250 °C erhitzt. Nach der vollständigen Abdestillation des entstandenen Phenols wird der Rückstand im Vakuum destilliert und aus Toluol umkristallisiert. Man erhält 490 g 1-Hydroxy-2,3-carbonyldioxybenzol (81 % d. Th.).

Diese werden in einem Gemisch von 25 ml konz. Schwefelsäure und 2,5 l Toluol gelöst. Bei Raumtemperatur wird diese Lösung innerhalb von 2 Stunden mit 496 g Methacrylsäureanhydrid versetzt und 12 Stunden lang bei Raumtemperatur gerührt. Die sich ergebende Mischung wird mehrere Male mit Wasser gewaschen. Die organische Phase wird vom Lösungsmittel befreit. Der verbliebene Rückstand wird nach Zugabe von Diisopropylether kristallin. Der kristalline Niederschlag wird abgesaugt und mit Diisopropylether gewaschen. Es verbleiben 475 g 1-Methacryloyloxy-2,3-carbonyldioxy-benzol (67 % d. Th.).

Diese werden 2 Stunden lang in 3 l Wasser bei 65°C gerührt. Unter Entwicklung von $CO_2$ entsteht eine klare Lösung. Nach dem Abkühlen werden die entstandenen Kristalle abgesaugt und aus Toluol umkristallisiert. Man erhält 385 g analysenreines 1,2-Dihydroxy-3-methacryloyloxy-benzol (92 % d. Th.) mit dem Schmelzpunkt 100 - 102 °C.

Die Gesamtausbeute, bezogen auf Pyrogallol, beträgt 50 % d. Th.

Beispiel 2

Herstellung von 1,2-Dihydroxy-3-acryloyloxy-benzol

In eine Mischung von 126 g Pyrogallol in 400 g Toluol und 160 g Pyridin leitet man unter Eiskühlung 99 g Phosgen ein. Nach 12 Stunden Rühren bei Raumtemperatur wird noch eine Stunde auf 100°C erwärmt. Nach dem Abkühlen wird das Reaktionsgemisch angesäuert und mit Wasser gewaschen. Das Lösungsmittel wird entfernt. Der Rückstand wird aus Toluol umkristallisiert. Man erhält 123 g 1-Hydroxy-2,3-carbonyldioxy-benzol (81 % d. Th.).

Diese werden in einem Gemisch von 81 g Triethylamin und 0,7 l Toluol gelöst. Bei Eiskühlung wird diese Lösung innerhalb von 3 Stunden mit 73 g Acrylsäurechlorid versetzt und 12 Stunden lang bei Raumtemperatur gerührt. Die organische Phase wird zunächst mit Wasser gewaschen und anschließend vom Lösungsmittel befreit. Der verbliebene Rückstand wird aus Diisopropylether umkristallisiert. Man erhält 130 g 1-Acryloyloxy-2,3-carbonyldioxybenzol (78 % d. Th.).

Diese werden 1,5 Stunden lang in 0,8 l Wasser bei 65°C gerührt. Unter $CO_2$-Entwicklung entsteht eine klare Lösung. Die nach dem Abkühlen ausgefallenen Kristalle werden abgesaugt und getrocknet. Man erhält 94 g 1,2-Dihydroxy-3-acryloyloxy-benzol (83 % d. Th.) mit dem Schmelzpunkt 92 - 94 °C.

Die Gesamtausbeute, bezogen auf Pyrogallol, beträgt 52 % d. Th.

Beispiel 3

Herstellung von 3,4-Dihydroxy-5-methacryloyloxy-benzoesäureethylester

In einem Kolben mit Destillationsaufsatz werden 198 g Gallussäureethylester und 204 g Diphenylcarbonat gut vermischt und allmählich auf 250°C erhitzt. Nach dem vollständigen Abdestillieren des entstandenen Phenols wird der Rückstand im Vakuum destilliert und aus Ethylacetat umkristallisiert. Man erhält 183 g 3-Hydroxy-4,5-carbonyldioxy-benzoesäureethylester (82 % d. Th.).

Diese werden in einem Gemisch aus 82 g Triethylamin in 800 ml Toluol gelöst. Bei Eiskühlung wird diese Lösung innerhalb von 3 Stunden mit 86 g Methacrylsäurechlorid versetzt und 12 Stunden lang bei Raumtemperatur gerührt. Die organische Phase wird zunächt mit Wasser gewaschen und anschließend vom Lösungsmittel befreit. Der verbliebene Rückstand wird aus Diisopropylether umkristallisiert. Man erhält 191 g 3-Methacryloyloxy-4,5-carbonyldioxy-benzoesäureethylester (80 % d. Th.).

Diese werden 5 Stunden lang in 1,2 l Wasser bei 65°C gerührt, bis eine klare Lösung entstanden ist. Nach dem Abkühlen werden die ausgefallenen Kristalle abgesaugt und aus Toluol umkristallisiert. Man erhält 146 g 3,4-Dihydroxy-5-methacryloyloxy-benzoesäureethylester (84 % d. Th.) mit dem Schmelzpunkt 106-108°C.

Die Gesamtausbeute, bezogen auf Gallussäureethylester, beträgt 55 % d. Th.

**Patentansprüche**

1. Verbindung der allgemeinen Formel I

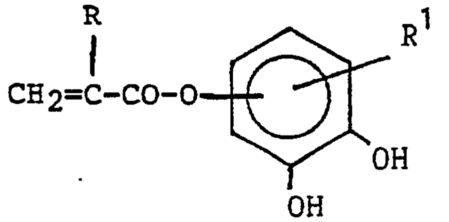

(I)

worin
R ein Wasserstoff- oder Halogenatom, eine Cyanid- oder eine Alkylgruppe mit 1-4 Kohlenstoffatomen und
$R^1$ ein Wasserstoff- oder Halogenatom, eine Nitro-, Alkyl-, Alkoxy-, Aryl-, Aryloxy-, Acyl- oder Alkoxycarbonylgruppe ist.

2. Verbindung nach Anspruch 1, dadurch gekennzeichnet, daß der Rest $CH_2$=CR-COO- in o-Stellung zu einer der OH-Gruppen steht.

3. Verfahren zur Herstellung von Verbindungen der Formel I gemäß Anspruch 1, dadurch gekennzeichnet, daß man eine Verbindung der Formel II

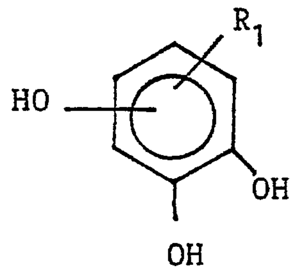

(II)

in der $R^1$ die in Anspruch 1 angegebene Bedeutung hat, mit einer Verbindung der Formel III

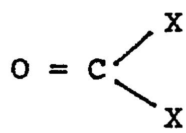

(III)

worin X ein Halogenatom, eine Alkoxy- oder Aryloxygruppe bedeutet, zu einer Verbindung der Formel IV

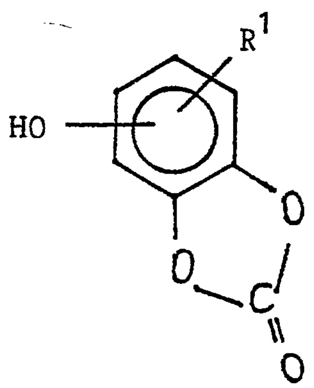

(IV)

umsetzt, die erhaltene Verbindung der Formel IV mit einer Verbindung der Formel V

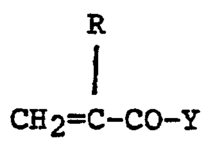

(V)

worin
Y ein Halogenatom, eine Hydroxygruppe, eine Alkoxygruppe oder ein Rest der Formel

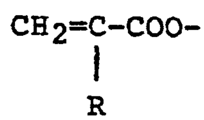

ist und
R die in Anspruch 1 angegebene Bedeutung hat, zu einer Verbindung der Formel VI

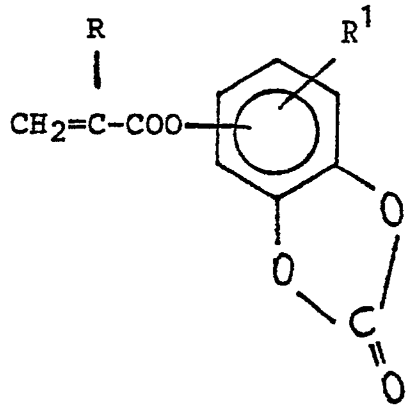

(VI)

umsetzt und die erhaltene Verbindung der Formel VI zu einer Verbindung der Formel I hydrolysiert.

**Claims**

1. A compound of the general formula I

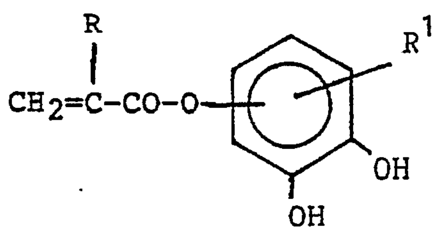

(I)

in which
R is a hydrogen or halogen atom, a cyanide or an alkyl group having 1–4 carbon atoms and
$R^1$ is a hydrogen or halogen atom, a nitro, alkyl, alkoxy, aryl, aryloxy, acyl or alkoxycarbonyl group.

2. The compound as claimed in claim 1, wherein the radical $CH_2$=CR–COO– is in the o-position relative to one of the OH groups.

3. A process for preparing compounds of the formula I as claimed in claim 1, wherein a compound of the formula II

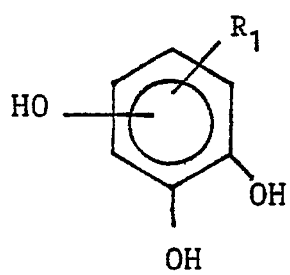

(II)

in which $R^1$ has the meaning indicated in claim 1, is reacted with a compound of the formula III

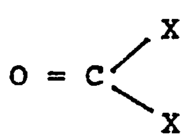

(III)

in which X denotes a halogen atom, an alkoxy or aryloxy group, to give a compound of the formula IV

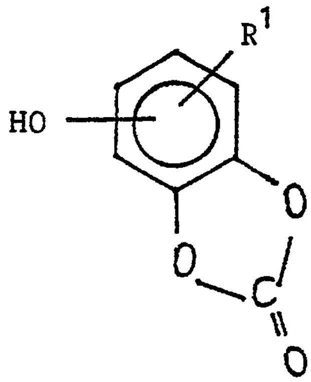

(IV)

the resulting compound of the formula IV is reacted with a compound of the formula V

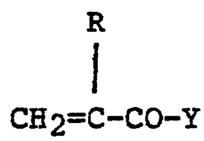

(V)

in which
Y is a halogen atom, a hydroxyl group, an alkoxy group or a radical of the formula

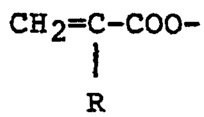

and
R has the meaning indicated in claim 1, to give a compound of the formula VI

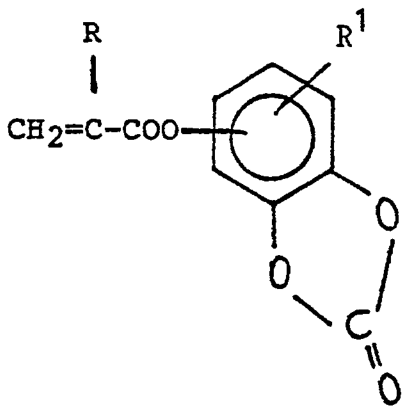

(VI)

and the resulting compound of the formula VI is hydrolized to a compound of the formula I.

**Revendications**

1. Composé de formule générale I

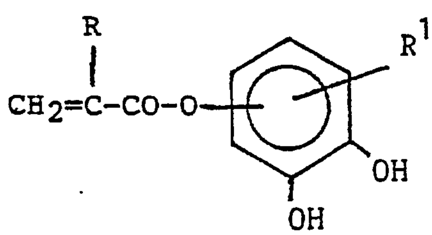

(I)

dans laquelle
R est un atome d'hydrogène ou d'halogène, un groupe cyano ou un groupe alkyle ayant de 1 à 4 atomes de carbone, et
$R^1$ est un atome d'hydrogène ou d'halogène, un groupe nitro, alkyle, alcoxy, aryle, aryloxy, acyle ou alcoxycarbonyle.

2. Composé selon la revendication 1, caractérisé en ce que le radical $CH_2$=CR-COO- se trouve en position ortho par rapport à l'un des groupes OH.

3. Procédé pour la préparation de composés de formule I selon la revendication 1, caractérisé en ce que l'on fait réagir un composé de formule II

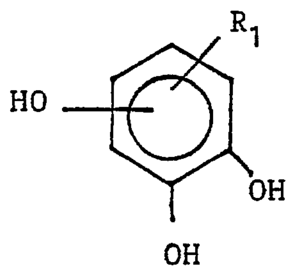

(II)

dans laquelle $R^1$ a la signification donnée dans la revendication 1, avec un composé de formule III

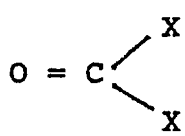

(III)

dans laquelle
X représente un atome d'halogène ou un groupe alcoxy ou aryloxy, pour aboutir à un composé de formule IV

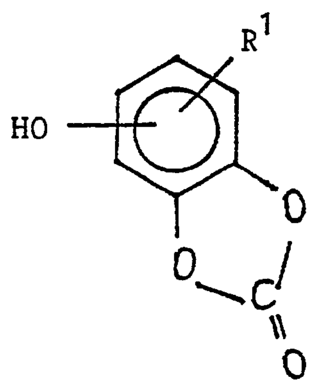

(IV)

à faire réagir le composé de formule IV obtenu avec un composé de formule V

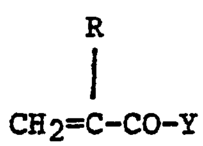

(V)

dans laquelle
Y représente un atome d'halogène ou un groupe hydroxy, un groupe alcoxy ou un radical de formule

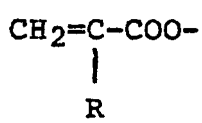

et R a la signification donnée dans la revendication 1, pour aboutir à un composé de formule VI

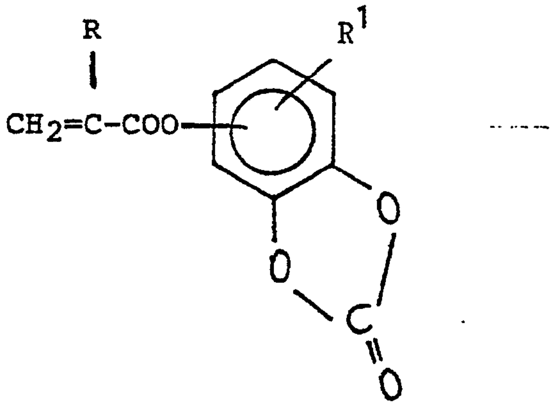

(VI)

et à hydrolyser le composé de formule VI obtenu pour aboutir à un composé de formule I.